Europäisches Patentamt

European Patent Office

Office européen des brevets

(11) Publication number: **0 085 579**
**B1**

(12) **EUROPEAN PATENT SPECIFICATION**

(45) Date of publication of patent specification: **06.05.87**

(21) Application number: **83300531.7**

(22) Date of filing: **02.02.83**

(51) Int. Cl.⁴: **A 61 K 45/06,** A 61 K 31/195,
A 61 K 31/20, A 61 K 31/375,
A 61 K 31/585, A 61 K 31/19,
A 61 K 31/61, A 61 K 31/355,
A 61 K 31/715, A 61 K 33/30,
A 61 K 35/78

(54) Topical pharmaceutical compositions.

(30) Priority: **03.02.82 US 345204**

(43) Date of publication of application:
**10.08.83 Bulletin 83/32**

(45) Publication of the grant of the patent:
**06.05.87 Bulletin 87/19**

(84) Designated Contracting States:
**AT BE CH DE FR GB IT LI LU NL SE**

(56) References cited:
**EP-A-0 068 854**
**US-A-3 639 625**
**US-A-4 328 243**

(73) Proprietor: **EFAMOL LIMITED**
**12th Floor Moor House London Wall**
**London EC2Y 5HE (GB)**

(72) Inventor: **Horrobin, David Frederick**
**P.O.Box 10 Nuns Island**
**Montreal H3E 1J8 (CA)**
Inventor: **Lieb, Julian**
**41 Village Lane**
**Bethany Connecticut 06525 (US)**

(74) Representative: **Cockbain, Julian Roderick**
**Michaelson et al ·**
**Frank B. Dehn & Co. European Patent Attorneys**
**Imperial House 15-19, Kingsway**
**London WC2B 6UZ (GB)**

Courier Press, Leamington Spa, England.

**0 085 579**

**Description**

This invention relates to topical pharmaceutical compositions and their use in the treatment of lesions of the skin and mucous membranes.

The oral and parenteral administration of lithium and in particular lithium salts such as lithium carbonate, has found widespread application in the treatment of manic-depressive psychosis. Lithium treatment has been reported as being particularly effective in the treatment of the manic phase of this illness and also in the prophylaxis of both manic and depressive relapses.

It has been reported (Lieb, N. Eng. J. Med. *301* (1979), 942) that the oral administration of lithium salts in the treatment of manic-depressive illness has been accompanied by the remission of recurrent herpes infection in a patient additionally suffering from labial herpes and in a patient also having genital herpes.

U.S. Patent Specification No. 3639625 (issued February 1972 to Sherwin) describes therapeutic compositions containing lithium succinate for treating dermatitis and for producing an antipruritic effect, the compositions thus being suitable for topical application. One of the compositions described also contains zinc phenosulphate and is described as being suitable for use in burns and abrasions.

U.S. Patent Specification No. 4328243 (issued May 1982 to Horrobin and Lieb) describes a method for the treatment of the side-effects of lithium treatment in a subject suffering from manic-depressive psychosis and undergoing lithium treatment by orally administering to the subject an effective amount of dihomo-γ-linolenic acid and/or γ-linolenic acid or linoleic acid.

European Patent Application Publication No. 68854 discloses oral compositions comprising a physiologically acceptable lithium salt together with at least one compound selected from dihomo-γ-linolenic acid, γ-linolenic acid and linoleic acid. These compositions are described as being useful in the treatment or prophylaxis of disorders of inflammation and immunity and disorders associated with smooth muscle spasm. The application claims priority inter alia from U.S. Patent Application No. 277671 which describes a method of treatment and/or prophylaxis of such disorders but does not describe any compositions containing a lithium salt with as a further component dihomo-γ-linolenic acid, γ-linolenic acid or linoleic acid.

We now propose that the conjoint topical administration of a lithium salt with one or more further substances selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, will be effective in the treatment of lesions of the skin and mucous membranes.

Thus, in one aspect, the invention provides a pharmaceutical composition for topical administration which comprises at least one physiologically acceptable lithium salt together with at least one further substance, said further substance being selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, said lithium salt being present in an amount sufficient to provide from 0.01 to 25% by weight of lithium ions in said composition.

Lesions of the skin and of mucous membranes are generally associated with an inflammatory response, and, in turn, inflammation is believed to be due in part to excessive and/or defective production of certain prostaglandins and related substances. Prostaglandins and related substances are biosynthesised in the body from two main substances, dihomo-γ-linolenic acid and arachidonic acid. In general, prostaglandins of the E-series exhibit a beneficial anti-inflammatory activity, and while minor amounts of such prostaglandins are formed from arachidonic acid, most of the prostaglandin products derived from arachidonic acid show an inflammatory action. The principal prostaglandin product derived from dihomo-γ-linolenic acid is prostaglandin E1 which exhibits the anti-inflammatory activity. Concomitant with the production of prostaglandins and related substances from dihomo-γ-linolenic acid and arachidonic acid by the cyclo-oxygenase enzyme, other bioproducts are formed from these acids as a result of the action of the enzyme, lipoxygenase. These lipoxygenase products also exhibit an inflammatory action.

Dihomo-γ-linolenic acid and arachidonic acid for metabolism in the body to prostaglandins and lipoxygenase products are usually available either from endogenous stores of these acids or from food sources. For example, dihomo-γ-linolenic acid may be biosynthesised from dietary sources of its precursor substances γ-linolenic acid and linoleic acid. In animals arachidonic acid may readily be biosynthesised from dihomo-γ-linolenic acid, but in adult humans such a mechanism is not particularly effective so that the major source of arachidonic acid for prostaglandin synthesis may be from ingestion of the acid *per se*.

We now believe that the previously noted activity of lithium succinate for treating dermatitis and for producing an anti-puritic effect is due to the ability of lithium to block the release of dihomo-γ-linolenic acid

and arachidonic acid from endogenous stores of these compounds, so that the availability of the compounds for conversion to the inflammatory prostaglandins and lipoxygenase products is reduced.

However, as indicated above, E-series prostaglandins are believed to be beneficial in the treatment of skin and mucous membrane lesions due to their anti-inflammatory activity, so that in one embodiment, the compositions according to the invention incorporate one or more substances which are capable of selectively increasing the *in vivo* level of E-series prostaglandins selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins and substances capable of blocking the bioconversion of arachidonic acid to prostaglandins.

For example, the *in vivo* level of E-series prostaglandins and especially prostaglandin $E_1$ may be increased by incorporating dihomo-γ-linolenic acid and/or its bioprecursors such as γ-linolenic acid and linoleic acid into the compositions, conveniently in an amount of from 0.01 to 80, preferably from 1 to 15, per cent by weight. If desired, the level of E-series prostaglandins may be increased by including in the composition a substance which is capable of activating the bioconversion of dihomo-γ-linolenic acid to E-series prostaglandins such as, for example, ascorbic acid (e.g. in an amount of from 0.01 to 20, preferably 0.1 to 5 per cent by weight), ethanol (e.g. in an amount of from 0.01 to 80, preferably 0.1 to 10% per cent, by weight) and spironolactone, (e.g. in an amount of from 0.01 to 20, preferably 0.1 to 5, per cent by weight).

The compositions of the invention may further include a substance which acts to mobilise the endogenous stores of dihomo-γ-linolenic acid and examples of such substances include physiologically acceptable zinc salts, which conveniently may be included in an amount sufficient to provide from 0.01 to 10, preferably 0.1 to 5, per cent by weight of zinc ions.

In the body, E-series prostaglandins may themselves act as bioprecursors for other prostaglandins. For example, prostaglandin $E_1$ may be converted to prostaglandin F1α, which does not· show the desired anti-inflammatory action. The level of E-series prostaglandins may therefore be increased by incorporating a substance which is capable of blocking their bioconversion to other prostaglandins. Examples of such substances are rutin and other bioflavanoids. Rutin may conveniently be incorporated into the compositions in an amount of from 0.01 to 20, preferably 0.1 to 10 per cent by weight.

As indicated above, prostaglandins of the E-series form only a minor proportion of the prostaglandin products of the metabolism of arachidonic acid. As the major proportion of the prostaglandin products from arachidonic acid do not provide an anti-inflammatory action, it may therefore be desirable to incorporate into the compositions of the invention a substance which is capable of selectively promoting the formation of E-series prostaglandins in the bioconversion of arachidonic acid. An example of a substance which may be used for this purpose is glutathione, conveniently in an amount of 0.01 to 20, preferably 0.1 to 5, per cent by weight of the composition. It may also be desired to include in the composition a substance which is capable of blocking the conversion of arachidonic acid to any prostaglandin. One such substance which may be used is (20:5n3) eicosapentaenoic acid and this may conveniently be present in an amount of from 0.01 to 20, preferably 0.1 to 5, per cent by weight.

As indicated the biosynthesis of prostaglandins from dihomo-γ-linolenic acid and arachidonic acid by the cyclo-oxygenase enzyme is also associated with the formation of lipoxygenase products which themselves exhibit inflammatory activity. Thus it has been found that the biosynthesis of the prostaglandins may be inhibited or blocked by substances which are able to inhibit the cyclo-oxygenase enzyme. It has also been found that the formation of lipoxygenase products may be inhibited or blocked e.g. in the presence of vitamin E and related tocopherols. Thus, in one embodiment, the compositions according to the invention may contain one or more substances which are capable of inhibiting the cyclo-oxygenase enzyme and/or one or more substances which are capable of inhibiting the formation of lipoxygenase products optionally in addition to one or more substances which are capable of selectively increasing the *in vivo* level of E-series prostaglandins. Substances which are capable of inhibiting the cyclo-oxygenase enzyme include, acetylsalicylic acid, indomethacin, mefenamic acid, ketoprofen, ibuprofen and paracetamol. The formation of lipoxygenase products may be inhibited by, vitamin E and/or related tocopherols, or any other physiologically acceptable lipoxygenase inhibitor.

Where the compositions according to the invention are to be used for topical application to lesions associated with viral infections, it may be desirable to incorporate lysine, which may be capable of inhibiting viral replication, into the compositions, optionally together with the substance capable of selectively increasing the *in vivo* level of E-series prostaglandins, the substance capable of inhibiting cyclooxygenase enzyme and/or the substance capable of inhibiting the formation of lipoxygenase products. Lysine may conveniently be present in the compositions in an amount of from 0.01 to 20, preferably 0.1 to 5, per cent by weight.

If oedema is present, this may limit the access of therapeutic agents to cells which are inflamed or infected. It may therefore be advantageous to administer the composition of the invention in a form which is capable of reducing local oedema and which will aid the penetration of the other components of the composition to the affected cells. This may be achieved by additionally incorporating one or more high molecular weight polysaccharides into the compositions. Examples of such polysaccharides include dextrans, such as dextran sulphate.

The compositions according to the invention are in a form suitable for topical administration. Examples of such forms include creams, ointments, solutions, suspensions, emulsions, lotions, gels and sprays. Such forms may be prepared with pharmaceutical carriers and excipients conventionally used for such purposes. The compositions of the invention are preferably in the form of ointments, which may conveniently be formulated using an appropriate base such as, for example, lanolin, paraffin or cetyl alcohol.

The compositions according to the invention may be used for the treatment of disorders of the skin and mucous membranes e.g. oral, nasal, ocular, aural and genital membranes. In particular, the compositions may be used in the treatment of pruritis, lesions arising from inflammatory disorders such as eczema and psoriasis and the lesions due to allergic reactions such as to poison ivy as well as having a soothing and analgesic effect on such lesions. In addition, the compositions may be used in the treatment of lesions resulting from viral infections, for example the lesions due to infection with herpes viruses such as herpes simplex or herpes zoster, e.g. in herpes labialis and genitalis, and shingles; as well as lesions arising from superficial wounds, burns, and local poisoning e.g. as a result of insect bites and stings.

In a further aspect, the invention provides the use of at least one physiologically acceptable lithium salt together with at least one substance selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, for the manufacture of a medicament for the treatment by topical administration of said medicament of lesions of the skin or mucous membrances of a subject.

In a yet further aspect, the invention provides a process for preparation of a pharmaceutical composition for topical administration which comprises admixing at least one physiologically acceptable lithium salt together with at least one further substance, said further substance being selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, said lithium salt being employed in an amount sufficient to provide from 0.01 to 25% by weight of lithium ions in said composition.

The lithium salts employed according to the invention will be physiologically acceptable, and examples of such salts include lithium carbonate, chloride, sulphate, citrate, succinate, salicylate and acetylsalicylate.

When the compositions according to the invention contain dihomo-γ-linolenic acid this may, if desired, be replaced, at least in part, by an equivalent amount of a biosynthetic precursor thereof such as the above-mentioned γ-linolenic acid or linoleic acid. If desired, these substances may be used in admixture. These substances may also be used in the form of physiologically acceptable functional derivatives thereof such as, for example, their $C_1$—$C_4$ alkyl (e.g. methyl and ethyl) esters and the triglycerides of the acids. Convenient sources of linoleic acid for use according to the invention are the many vegetable oils of which it forms a major constituent. Examples of such oils include cotton-seed, soyabean, peanut, corn, sunflower seed, safflower, poppy seed, linseed and perilla oils, where the linoleic acid occurs in the form of its triglyceride, and the vegetable oils may be used as such i.e. without any treatment to isolate the linoleic acid therefrom.

At the present time known sources of oils having a high γ-linolenic acid content are few. One source currently available is the seed of the Evening Primrose or *Oenothera biennis L*, the oil extract therefrom containing γ-linolenic acid and linoleic acid in the form of their triglycerides. Another source of γ-linolenic acid is the seed of *Borago officinalis* which provides a richer source of γ-linolenic acid with smaller amounts of linoleic acid. Again, these seed oil extracts may be used as such or may, if desired, be fractionated to yield an oil composition enriched in the desired γ-linolenic and/or linoleic acids.

Dihomo-γ-linolenic acid for use according to the invention may be prepared from γ-linolenic acid according to known methods.

If convenient, it may be appropriate to utilise the lithium in the form of a salt with the above mentioned acids, that is with dihomo-γ-linolenic, γ-linolenic or linoleic acid.

The bioconversion of linoleic acid to γ-linolenic acid, which is itself subsequently converted to dihomo-γ-linolenic acid, is promoted in the presence of zinc. We have found that the conversion of dihomo-γ-linolenic acid to prostaglandin E1 is also enhanced by zinc. Thus, as indicated above, the compositions according to the invention may if desired contain a physiologically acceptable zinc salt such as, for example, zinc sulphate or gluconate. The use of a zinc salt in compositions of the invention may be beneficial independent of its effects on fatty acid and prostaglandin metabolism, as it may have healing properties of its own.

Compositions according to the invention contain an amount of lithium salt sufficient to provide from 0.01 to 25, preferably 1 to 5, per cent by weight of lithium ions in the compositions. When the composition

4

contains vitamin E and/or related tocopherols, these are conveniently present in an amount of 0.01 to 25, preferably 1 to 10, per cent by weight.

Under certain circumstances, it may be desirable to limit the formation of all cyclo-oxygenase and lipoxygenase products from both dihomo-γ-linolenic acid and arachidonic acid. In this situation it may be appropriate to use a combination of a lithium salt and a tocopherol which is capable of inhibiting the formation of lipoxygenase products without conjointly administering dihomo-γ-linolenic acid or its precursors. The following Examples serve to illustrate the invention:

Example 1
Ointment

| | % By weight |
|---|---|
| Lithium citrate | 8 |
| Vitamin E | 1 |
| Oil of Evening Primrose | 8 |
| Zinc sulphate | 2 |
| Dextran sulphate | 2 |

The above components are formulated with an appropriate ointment base, such as a base containing one or more cetyl alcohols with non-irritant emulsifiers or a lanolin base.

Examples 2—8
Ointments

The following components are formulated in the amounts shown in Table I with an appropriate ointment base, such as those described in Example 1:—

TABLE I

| | Ex. 2 | Ex. 3 | Ex. 4 | Ex. 5 | Ex. 6 | Ex. 7 | Ex. 8 |
|---|---|---|---|---|---|---|---|
| | | | % By weight | | | | |
| Lithium succinate | 6 | 8 | — | — | — | 8 | 8 |
| Lithium citrate | — | — | — | 8 | 8 | — | — |
| Lithium acetyl salicylate | — | — | 5 | — | — | — | — |
| Vitamin E | 1 | 1 | 1 | 1 | 1 | 1 | 1 |
| Oil of Evening Primrose | 4 | — | — | 8 | 5 | 5 | — |
| Zinc sulphate | 2 | — | — | 2 | — | — | 2 |
| Dextran sulphate | 2 | 2 | 3 | — | — | — | 2 |

Examples 9—11
Ointments

The following components are formulated in the per cent by weight amounts shown in Table II with an appropriate ointment base, such as those described in Example 1:—

TABLE II

| | Ex. 9 | Ex. 10 | Ex. 11 |
|---|---|---|---|
| Lithium succinate | 8 | — | — |
| Lithium citrate | — | 6 | 5 |
| Vitamin E | — | 1 | 2 |
| Indomethacin | 1 | — | 2 |
| Mefenamic acid | — | 2 | — |

Examples 12—25

Ointments

The following components are formulated in the per cent by weight amounts shown in Table III with an appropriate ointment base, such as those described in Example 1:—

TABLE III

| | Ex. 12 | Ex. 13 | Ex. 14 | Ex. 15 | Ex. 16 | Ex. 17 | Ex. 18 |
|---|---|---|---|---|---|---|---|
| Lithium succinate | 8 | 4 | — | — | 5 | — | — |
| Lithium citrate | — | — | 6 | 8 | — | 6 | — |
| Lithium acetylsalicylate | — | — | — | — | — | — | 5.0 |
| Indomethacin | — | 0.5 | 1.0 | — | — | — | — |
| Vitamin E | 1.0 | 0.5 | 0.5 | 1.0 | 0.5 | 1.0 | 1.0 |
| Evening primrose oil | 2.0 | 1.0 | — | 2.0 | — | — | 3.0 |
| Dihomo-γ-linolenic acid | — | — | 2.0 | — | — | 3.0 | — |
| Zinc sulphate | 0.05 | 0.1 | 0.2 | — | 0.05 | — | — |
| Dextran sulphate | 2.0 | 2.0 | 3.0 | — | — | 1.0 | — |
| Spironolactone | 1.0 | 1.0 | 0.5 | — | 0.5 | — | — |
| Ascorbic acid | 1.0 | 1.0 | 2.0 | — | — | 2.0 | — |
| Ethanol | 5.0 | 5.0 | 4.0 | — | 3.0 | — | 5.0 |
| Eicosapentaenoic acid | 1.0 | 1.0 | 1.0 | — | — | 5.0 | — |
| Glutathione | 1.0 | 1.0 | 0.5 | — | 1.0 | 0.5 | 1.0 |
| Rutin | 0.5 | 1.0 | 0.5 | — | — | — | 0.5 |
| Lysine | 0.5 | 1.0 | 2.0 | — | — | 2.0 | 1.0 |

TABLE III (continued)

| | Ex. 19 | Ex. 20 | Ex. 21 | Ex. 22 | Ex. 23 | Ex. 24 | Ex. 25 |
|---|---|---|---|---|---|---|---|
| Lithium succinate | — | 8.0 | — | — | 6.0 | — | 7.0 |
| Lithium citrate | — | — | 4.0 | — | — | 5.0 | — |
| Lithium acetylsalicylate | 10.0 | — | — | 4.0 | — | — | — |
| Indomethacin | — | 1.0 | — | — | — | — | — |
| Vitamin E | 1.0 | — | — | — | — | — | — |
| Evening primrose oil | — | — | 2.0 | — | — | — | — |
| Dihomo-γ-linolenic acid | — | — | — | — | — | — | — |
| Zinc sulphate | 0.5 | — | — | — | — | — | — |
| Dextran sulphate | — | — | — | — | — | — | — |
| Spironolactone | — | — | — | — | 1.0 | — | — |
| Ascorbic acid | 1.0 | — | — | — | — | — | — |
| Ethanol | — | — | — | — | — | — | — |
| Eicosapentaenoic acid | — | — | — | 2.0 | — | — | — |
| Glutathione | 2.0 | — | — | — | — | — | 2.0 |
| Rutin | — | — | — | — | — | — | — |
| Lysine | 0.5 | — | — | — | — | 1.0 | — |

**Claims for the Contracting States: BE, CH, DE, FR, GB, IT, LI, LU, NL, SE**

1. A pharmaceutical composition for topical administration which comprises at least one physiologically acceptable lithium salt together with at least one further substance, said further substance being selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, said lithium salt being present in a concentration sufficient to provide from 0.01 to 25% by weight of lithium ions in said composition.

2. A composition according to claim 1 containing at least one substance selected from dihomo-γ-linolenic acid, γ-linolenic acid and linoleic acid.

3. A composition according to claim 1 containing a substance capable of activating the bioconversion of dihomo-γ-linolenic acid to E-series prostaglandins selected from ascorbic acid, ethanol and spironolactone.

4. A composition according to claim 1 containing, as a substance capable of blocking the bioconversion of E-series prostaglandins to other prostaglandins, rutin.

5. A composition according to claim 1 containing, as a substance capable of promoting the bioconversion of arachidonic acid to E-series prostaglandins, glutathione.

6. A composition according to claim 1 containing, as a substance capable of blocking the bioconversion of arachidonic acid to prostaglandins, (20:5n3) eicosapentaenoic acid.

7. A composition according to claim 1 containing a substance capable of inhibiting cyclooxygenase enzyme selected from acetylsalicyclic acid, indomethacin, mefenamic acid, ketoprofen, ibuprofen and paracetamol.

8. A composition according to claim 1 containing a substance capable of inhibiting the *in vivo* formation of lipoxygenase products selected from vitamin E and related tocopherols.

9. A composition according to claim 1 further containing a physiologically acceptable zinc salt.

10. A composition according to claim 1 which additionally contains a dextran.

11. The use of at least one physiologically acceptable lithium salt together with at least one substance selected from dihomo-γ-linolenic acid and bioprecursors therefor, substances capable of activating the bioconversion of dihomo-γ-linolenic acid to E-series prostaglandins, substances capable of blocking the bioconversion. of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, for the manufacture of a medicament for the treatment by topical. administration of said medicament of lesions of the skin or mucous membranes of a subject.

12. Use as claimed in claim 11 of at least one physiologically acceptable lithium salt and at least one substance selected from dihomo-γ-linolenic acid, γ-linolenic acid and linoleic acid for the manufacture of said medicament.

**Claims for the Contracting State: AT**

1. A process for preparation of a pharmaceutical composition for topical administration which comprises admixing at least one physiologically acceptable lithium salt together with at least one further substance, said further substance being selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, said lithium salt being employed in an amount sufficient to provide from 0.01 to 25% by weight of lithium ions in said composition.

2. A process as claimed in claim 1 wherein said at least one further substance is selected from dihomo-γ-linolenic acid, γ-linolenic acid and linoleic acid.

3. A process as claimed in claim 1 wherein said at least one further substance is selected from ascorbic acid, ethanol and spironolactone.

4. A process as claimed in claim 1 wherein said at least one further substance is rutin.

5. A process as claimed in claim 1 wherein said at least one further substance is glutathione.

6. A process as claimed in claim 1 wherein said at least one further substance is (20:5n3) eicosapentaenoic acid.

7. A process as claimed in claim 1 wherein said at least one further substance is selected from acetylsalicyclic acid, indomethacin, mefenamic acid, ketoprofen, ibuprofen and paracetamol.

8. A process as claimed in claim 1 wherein said at least one further substance is selected from vitamin E and related tocopherols.

9. A process as claimed in any one of claims 1 to 8 wherein there is admixed a physiologically acceptable zinc salt together with said at least one physiologically acceptable lithium salt and said at least one further substance.

10. A process as claimed in any one of claims 1 to 9 wherein there is admixed a dextran together with said at least one physiologically acceptable lithium salt and said at least one further substance.

11. The use of at least one physiologically acceptable lithium salt together with at least one substance selected from dihomo-γ-linolenic acid and bioprecursors therefor, substances capable of activating the bioconversion of dihomo-γ-linolenic acid to E-series prostaglandins, substances capable of blocking the bioconversion of E-series prostaglandins to other prostaglandins, substances capable of promoting the bioconversion of arachidonic acid to E-series prostaglandins, substances capable of blocking the bioconversion of arachidonic acid to prostaglandins, substances capable of inhibiting cyclooxygenase enzyme, substances capable of inhibiting the formation of lipoxygenase products, and lysine, for the manufacture of a medicament for the treatment by topical administration of said medicament of lesions of the skin or mucous membranes of a subject.

12. Use as claimed in claim 11 of at least one physiologically acceptable lithium salt and at least one substance selected from dihomo-γ-linolenic acid, γ-linolenic acid and linoleic acid for the manufacture of said medicament.

**Patentansprüche für die Vertragsstaaten: BE, CH, DE, FR, GB, IT, LI, LU, NL und SE**

1. Pharmazeutisches Mittel zur topischen Verabreichung, umfassend wenigstens ein physiologisch verträgliches Lithium-Salz zusammen mit wenigstens einer weiteren Substanz, wobei die weitere Substanz ausgewählt ist unter Dihomo-γ-linolensäure und deren Bio-Precursoren; Substanzen, die in der Lage sind, die Bio-Umwandlung von Dihomo-γ-linolensäure in Prostaglandine der E-Reihe zu aktivieren; Substanzen, die in der Lage sind, die Bio-Umwandlung der Prostaglandine der E-Reihe in andere Prostaglandine zu blockieren; Substanzen, die in der Lage sind, die Bio-Umwandlung der Arachidonsäure in Prostaglandine der E-Reihe zu fördern; Substanzen, die in der Lage sind, die Bio-Umwandlung der Arachidonsäure in Prostaglandine zu blockieren; Substanzen, die in der Lage sind, das Cyclooxygenase-Enzym zu inhibieren;

8

**0 085 579**

Substanzen, die in der Lage sind, die Bildung von Lipoxygenase-Produkten zu hemmen und Lysin, wobei das Lithium-Salz in einer Konzentration vorhanden ist, die ausreicht, um 0,01—25 Gew.-% Lithium-Ionen in dem Mittel zur Verfügung zu stellen.

2. Mittel nach Anspruch 1, enthaltend wenigstens eine Substanz ausgewählt unter Dihomo-γ-linolensäure, γ-Linolensäure und Linolsäure.

3. Mittel nach Anspruch 1, enthaltend eine Substanz, die in der Lage ist, die Bio-Umwandlung von Dihomo-γ-linolensäure in Prostaglandine der E-Reihe zu aktivieren, ausgewählt unter Ascorbinsäure, Äthanol und Spironolacton.

4. Mittel nach Anspruch 1, enthaltend Rutin als Substanz, die in der Lage ist, die Bio-Umwandlung der Prostaglandine der E-Reihe in andere Prostaglandine zu blockieren.

5. Mittel nach Anspruch 1, enthaltend Glutathion als Substanz, die in der Lage ist, die Bio-Umwandlung von Arachidonsäure in Prostaglandine der E-Reihe zu fördern.

6. Mittel nach Anspruch 1, enthaltend (20:5n3) Eikosapentaensäure, als Substanz, die in der Lage ist, die Bio-Umwandlung der Arachidonsäure in Prostaglandine zu blockieren.

7. Mittel nach Anspruch 1, enthaltend eine Substanz, die in der Lage ist, das Cyclooxygenase-Enzym zu inhibieren, ausgewählt unter Acetylsalicylsäure, Indomethacin, Mefenaminsäure, Ketoprofen, Ibuprofen und Paracetamol.

8. Mittel nach Anspruch 1, enthaltend eine Substanz, die in der Lage ist, die in vivo Bildung von Lipoxygenase-Produkten zu inhibieren, ausgewählt unter Vitamin E under verwandten Tocopherolen.

9. Mittel nach Anspruch 1, zusätzlich enthaltend ein physiologisch verträgliches Zinksalz.

10. Mittel nach Anspruch 1, das zusätzlich ein Dextran enthält.

11. Verwendung von wenigstens einem physiologisch verträglichen Lithium-Salz, zusammen mit mindestens einer Substanz ausgewählt unter Dihomo-γ-Linolensäure und deren Bio-Precursoren; Substanzen, die in der Lage sind, die Bio-Umwandlung von Dihomo-γ-linolensäure in Prostaglandine der E-Reihe zu aktivieren; Substanzen, die in der Lage sind, die Bio-Umwandlung von Prostaglandinen der E-Reihe in andere Prostaglandine zu blockieren; Substanzen, die in der Lage sind, die Bio-Umwandlung der Arachidonsäure in Prostaglandine der E-Reihe zu fördern; Substanzen, die in der Lage sind, die Bio-Umwandlung der Arachidonsäure in Prostaglandine zu blockieren; Substanzen, die in der Lagen sind, das Cyclooxygenase-Enzym zu inhibieren; Substanzen, die in der Lage sind, die Bildung der Lipoxygenase-Produkte zu inhibieren und Lysin, zur Herstellung eines Mittels zur Behandlung von Läsionen der Haut und Schleimhaut eines Individuums durch topische Verabreichung des Mittels.

12. Verwendung gemäß Anspruch 11 von mindestens einem physiologisch verträglichen Lithium-Salz und mindestens einer Substanz, ausgewählt unter Dihomo-γ-linolensäure, γ-Linolensäure und Linolsäure zur Herstellung des Mittels.

**Patentansprüche für den Vertragsstaat: AT**

1. Verfahren zur Herstellung eines pharmazeutischen Mittels zur topischen Verabreichung, wobei man mindestens ein physiologisch verträgliches Lithiumsalz mit mindestens einer weiteren Substanz vermischt, wobei die weitere Substanz ausgewählt ist unter Dihomo-γ-linolensäure und deren Bio-Precursoren; Substanzen, die in der Lage sind, die Bio-Umwandlung von Dihomo-γ-linolensäure in Prostaglandine der E-Reihe zu aktivieren; Substanzen, die in der Lage sind, die Bio-Umwandlung der Prostaglandine der E-Reihe in andere Prostaglandine zu blockieren; Substanzen, die in der Lage sind, die Bio-Umwandlung der Arachidonsäure in Prostaglandine der E-Reihe zu fördern; Substanzen, die in der Lage sind, die Bio-Umwandlung der Arachidonsäure zu Prostaglandinen zu blockieren; Substanzen, die in der Lage sind, das Cyclooxygenase-Enzym zu inhibieren; Substanzen, die in der Lage sind, die Bildung von Lipoxygenase-Produkte zu inhibieren und Lysin, wobei das Lithium-Salz in einer Menge verwendet wird, die ausreicht, um 0,01—25 Gew.-% Lithium-Ionen in dem Mittel zur Verfügung zu stellen.

2. Verfahren nach Anspruch 1, worin die weitere Substanz ausgewählt ist unter Dihomo-γ-linolensäure, γ-Linolen-Säure und Linolsäure.

3. Verfahren nach Anspruch 1, worin die weitere Substanz ausgewählt ist unter Ascorbinsäure, Äthanol und Spironolacton.

4. Verfahren nach Anspruch 1, worin die weitere Substanz Rutin ist.

5. Verfahren nach Anspruch 1, worin die weitere Substanz Glutathion ist.

6. Verfahren nach Anspruch 1, worin die weitere Substanz (20:5n3) Eicosapentaensäure ist.

7. Verfahren nach Anspruch 1, worin die weitere Substanz ausgewählt ist unter Acetylsalicylsäure, Indomethacin Mefenaminsäure, Ketoprofen, Ibuprofen und Paracetamol.

8. Verfahren nach Anspruch 1, worin die weitere Substanz ausgewählt ist unter Vitamine E und verwandten Tocopherolen.

9. Verfahren nach einem der Ansprüche 1 bis 8, worin ein physiologisch verträgliches Zink-Salz zusammen mit mindestens einem physiologisch verträglichen Lithium-Salz und mindestens einer weiteren Substanz vermischt wird.

10. Verfahren nach einem der Ansprüche 1 bis 9, worin ein Dextran zusammen mit mindestens einem physiologisch verträglichen Lithium-Salz und mindestens einer weiteren Substanz vermischt wird.

11. Verwendung von mindestens einem physiologisch verträgliche Lithium-Salz zusammen mit

9

**0 085 579**

mindestens einer weiteren Substanz, ausgewählt unter Dihomo-γ-Linolen-Säure und deren Bio-Precursoren; Substanzen, die in der Lage sind, die Bio-Umwandlung von Dihomo-γ-Linolensäure in Prostaglandine der E-Reihe zu aktivieren; Substanzen, die in der Lage sind, die Bio-Umwandlung von Prostaglandinen der E-Reihe in andere Prostaglandine zu blockieren; Substanzen, die in der Lage sind, die Bio-Umwandlung der Arachidonsäure in Prostaglandine der E-Reihe zu fördern; Substanzen, die in der Lage sind, die Bio-Umwandlung der Arachidonsäure in Prostaglandine zu blockieren; Substanzen, die in der Lage sind, das Cyclooxygenase-Enzym zu inhibieren; Substanzen, die in der Lage sind, die Bildung der Lipoxygenase-Produkte zu inhibieren und Lysin, zur Herstellung eines Mittels zur Behandlung von Läsionen der Haut und Schleimhaut eines Individuums durch topische Verabreichung des Mittels.

12. Verwendung gemäß Anspruch 11 von mindestens einem physiologisch verträglichen Lithium-Salz und mindestens einer weiteren Substanz, ausgewählt unter Dihomo-γ-linolensäure, γ-Linolensäure und Linolsäure, zur Herstellung des Mittels.

**Revendications pour les Etats Contractants: BE, CH, DE, FR, GB, IT, LI, LU, NL, SE**

1. Composition pharmaceutique destinée à l'administration par voie topique, caractérisée en ce qu'elle comprend au moins un sel de lithium physiologiquement acceptable, ainsi qu'au moins une substance complémentaire, laquelle substance complémentaire est choisie parmi l'acide dihomo-γ-linolénique et les bioprécurseurs de ce dernier composé, des substances capables d'activer la bioconversion de l'acide dihomo-γ-linolénique en prostaglandines de la série E, des substances capable de bloquer la bioconversion de prostaglandines de la série E en d'autres prostaglandines, des substances susceptibles de promouvoir la bioconversion de l'acide arachidonique en prostaglandines de la série E, des substances capables de bloquer la bioconversion de l'acide arachidonique en prostaglandines, des substances capables d'inhiber l'enzyme qu'est la cyclooxygénase, des substances capables d'inhiber la formation de produits de la lipoxygénase, et de la lysine, le sel de lithium précité étant présent en une concentration qui suffit à constituer de 0,01 à 25% en poids d'ions lithium dans la composition précitée.

2. Composition suivant la revendication 1, caractérisée en ce qu'elle contient au moins une substance choisie parmi l'acide dihomo-γ-linolénique, l'acide γ-linolénique et l'acide linoléique.

3. Composition suivant la revendication 1, caractérisée en ce qu'elle contient une substance capable d'activer la bioconversion de l'acide dihomo-γ-linolénique en prostaglandines de la série E, choisie parmi l'acide ascorbique, l'éthanol et la spironolactone.

4. Composition suivant la revendication 1, caractérisée en ce qu'elle contient de la rutine à titre de substance capable de bloquer la bioconversion de prostaglandines de la série E en d'autres prostaglandines.

5. Composition suivant la revendication 1, caractérisée en ce qu'elle contient du glutathion à titre de substance capable de promouvoir la bioconversion de l'acide arachidonique en prostaglandines de la série E.

6. Composition suivant la revendication 1, caractérisée en ce qu'elle contient de l'acide (20:5n3) eicosapentaénoïque à titre de substance capable de bloquer la bioconversion de l'acide arachidonique en prostaglandines.

7. Composition suivant la revendication 1, caractérisée en ce qu'elle contient une substance capable d'inhiber l'enzyme qu'est la cyclooxygénase, choisie parmi l'acide acétylsalicylique, l'indométhacine l'acide méfénamique, le cétoprofen, l'ibuprofen et le paracétamol.

8. Composition suivant la revendication 1, caractérisée en ce qu'elle contient une substance capable d'inhiber la formation *in vivo* de produits de lipoxygénase choisis parmi la vitamine E et les tocophérols apparentés.

9. Composition suivant la revendication 1, caractérisée en ce qu'elle contient en outre un sel de zinc physiologiquement acceptable.

10. Composition suivant la revendication 1, caractérisée en ce qu'elle contient complémentairement un dextrane.

11. Utilisation d'au moins un sel de lithnium physiologiquement acceptable en même temps qu'au moins une substance choisie parmi l'acide dihomo-γ-linolénique et les bioprécurseurs de ce composé, des substances capables d'activer la bioconversion de l'acide dihomo-γ-linolénique en prostaglandines de la série E, des substances capables de bloquer la bioconversion de prostaglandines de la série E en d'autres prostaglandines, des substances capables de promouvoir la bioconversion de l'acide arachidonique en prostaglandines de la série E, des substances capables de bloquer la bioconversion de l'acide arachidonique en prostaglandines, des substances capables d'inhiber l'enzyme qu'est la cyclooxygénase, des substances capables d'inhiber la formation de produits de lipoxygénase, et de la lysine, en vue de la fabrication d'un médicament destiné au traitement de lésions de la peau ou de muqueuses d'un sujet par administration topique du médicament en question.

12. Utilisation suivant la revendication 11 d'au moins un sel de lithium physiologiquement acceptable et d'au moins une substance choisie parmi l'acide dihomo-γ-linolénique, l'acide γ-linolénique et l'acide linoléique en vue de la fabrication du médicament en question.

**Revendications pour l'Etat Contractant: AT**

1. Procédé de préparation d'une composition pharmaceutique destinée à l'administration par la voie topique, caractérisé en ce que l'on mélange au moins un sel de lithium physiologiquement acceptable ainsi qu'au moins une substance complémentaire, ladite substance complémentaire étant choisie parmi l'acide dihomo-γ-linolénique et les bioprécurseurs de ce composé, des substances capables d'activer la bioconversion de l'acide dihomo-γ-linolénique en prostaglandines de la série E, des substances capables de bloquer la bioconversion de prostaglandines de la série E en d'autres prostaglandines, des substances capables de promouvoir la bioconversion de l'acide arachidonique en prostaglandines de la série E, des substances capables de bloquer la bioconversion de l'acide arachidonique en prostaglandines, des substances capables d'inhiber l'enzyme qu'est la cyclooxygénase, des substances capables d'inhiber la formation de produits de lipoxygénase, et de la lysine, le sel de lithium précité étant utilisé en une quantité qui suffit à constituer de 0,01 à 25% en poids d'ions lithium dans ladite composition.

2. Procédé suivant la revendication 1, caractérisé en ce qu'au moins ladite substance complémentaire est choisie parmi l'acide dihomo-γ-linolénique, l'acide γ-linolénique et l'acide linoléique.

3. Procédé suivant la revendication 1, caractérisé en ce que l'on choisit au moins ladite substance complémentaire parmi l'acide ascorbique, l'éthanol et la spironolactone.

4. Procédé suivant la revendication 1, caractérisé en ce qu'au moins ladite substance complémentaire est la rutine.

5. Procédé suivant la revendication 1, caractérisé en ce qu'au moins ladite substance complémentaire est le glutathion.

6. Procédé suivant la revendication 1, caractérisé en ce qu'au moins ladite substance complémentaire est l'acide (20:5n3) éicosapentaénoïque.

7. Procédé suivant la revendication 1, caractérisé en ce qu'au moins ladite substance complémentaire est choisie parmi l'acide acétylsalicylique, l'indométhacine, l'acide méfénamique, le cétoprofen, l'ibuprofen et le paracétamol.

8. Procédé suivant la revendication 1, caractérisé en ce qu'au moins ladite substance complémentaire est choisie parmi la vitamine E et les tocophérols apparentés.

9. Procédé suivant l'une quelconque des revendications 1 à 8, caractérisé en ce que l'on mélange un sel de zinc physiologiquement acceptable à au moins ledit sel de lithium physiologiquement acceptable et au moins à une substance supplémentaire.

10. Procédé suivant l'une quelconque des revendications 1 à 9, caractérisé en ce que l'on mélange un dextrane à au moins ledit sel de lithium physiologiquement acceptable et à au moins une substance supplémentaire.

11. Utilisation d'au moins un sel de lithium physiologiquement acceptable en même temps qu'au moins une substance choisie parmi l'acide dihomo-γ-linolénique et les bioprécurseurs de ce composé, des substances capables d'activer la bioconversion de l'acide dihomo-γ-linolénique en prostaglandines de la série E, des substances capables de bloquer la bioconversion de prostaglandines de la série E en d'autres prostaglandines, des substances capables de promouvoir la bioconversion de l'acide arachidonique en prostaglandines de la série E, des substances capables de bloquer la bioconversion de l'acide arachidonique en prostaglandines, des substances capables d'inhiber l'enzyme qu'est la cyclooxygénase, des substances capables d'inhiber la formation de produits de lipoxygénase, et de la lysine, en vue de la fabrication d'un médicament destiné au traitement de lésions de la peau ou de muqueuses d'un sujet par administration topique du médicament en question.

12. Utilisation suivant la revendication 11 d'au moins un sel de lithium physiologiquement acceptable et d'au moins une substance choisie parmi l'acide dihomo-γ-linolénique, l'acide γ-linolénique et l'acide linoléique en vue de la fabrication du médicament en question.